(19) Europäisches Patentamt
European Patent Office
Office européen des brevets

(11) EP 2 886 101 A1

# (12) EUROPEAN PATENT APPLICATION

(43) Date of publication:
**24.06.2015 Bulletin 2015/26**

(21) Application number: **14198419.5**

(22) Date of filing: **17.12.2014**

(51) Int Cl.:
***A61K 8/86*** *(2006.01)* ***A61Q 17/04*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.12.2013 US 201314132290**

(71) Applicant: **JOHNSON & JOHNSON CONSUMER COMPANIES, INC.**
**Skillman,**
**New Jersey 08558 (US)**

(72) Inventors:
- **Daly, Susan**
  **Basking Ridge, NJ New Jersey 07920 (US)**
- **Fevola, Michael J.**
  **Belle Mead, NJ New Jersey 08502 (US)**
- **Tokgoz-Engrand, Selcan**
  **75005 Paris (FR)**

(74) Representative: **Carpmaels & Ransford LLP**
**One Southampton Row**
**London WC1B 5HA (GB)**

## (54) Sunscreen compositions containing an ultraviolet radiation-absorbing polymer

(57) Polymer compositions containing an ultraviolet radiation absorbing polyglycerol that has low fractions of diglycerol chromophore conjugates and that includes a UV-chromophore chemically bound thereto are provided, as well as processes to prepare such polymer compositions that includes preparing a polyglycerol intermediate by polymerizing glycerol; removing residual glycerol and low molecular weight fractions from the polyglycerol intermediate to form an enriched polyglycerol intermiedate having low fractions of diglycerol; and reacting the enriched polyglycerol intermediate with a UV-chromophore having a complementary functional group to form the polymer composition, and compositions using the ultraviolet radiation absorbing polyglycerol.

EP 2 886 101 A1

**Description**

## FIELD OF THE INVENTION

The present invention relates to polymer compositions comprising UV-absorbing polyglycerols.

## BACKGROUND OF THE INVENTION

The prolonged exposure to ultraviolet (UV) radiation, such as from the sun, can lead to the formation of light dermatoses and erythemas, as well as increase the risk of skin cancers, such as melanoma, and accelerate skin aging, such as loss of skin elasticity and wrinkling.

Numerous sunscreen compositions are commercially available with varying ability to shield the body from ultraviolet light. However, numerous challenges still exist to provide sunscreen compositions that provide strong UV radiation protection.

## SUMMARY OF THE INVENTION

The present invention relates to polymer compositions including an ultraviolet radiation absorbing polyglycerol. The ultraviolet radiation absorbing polyglycerol includes a UV-chromophore chemically bound thereto. The polymer composition has low fractions of diglycerol chromophore conjugates. The present invention further relates to processes for making the ultraviolet radiation absorbing polyglycerol that includes preparing a polyglycerol intermediate by reacting glycerol with a multivalent inorganic base; and reacting the polyglycerol intermediate with a UV-chromophore having a complementary functional group to form the polymer composition.

## BRIEF DESCRIPTION OF THE FIGURES

Figure 1 is a chromatogram of a polymer composition of the present invention.
Figure 2 is a chromatogram of a comparative polymer composition.

## DETAILED DESCRIPTION OF THE INVENTION

Unless defined otherwise, all technical and scientific terms used herein have the same meaning as commonly understood by one of ordinary skill in the art to which the invention belongs. As used herein, unless otherwise indicated, all hydrocarbon groups (e.g., alkyl, alkenyl) groups may be straight or branched chain groups. As used herein, unless otherwise indicated, the term "molecular weight" refers to weight average molecular weight, (Mw).

Unless defined otherwise, all concentrations refer to concentrations by weight of the composition. Also, unless specifically defined otherwise, the term "essentially free of," with respect to a class of ingredients, refers to the particular ingredient(s) being present in a concentration less than is necessary for the particularly ingredient to be effective to provide the benefit or property for which it otherwise would be used, for example, about 1% or less, or about 0.5% or less.

As used herein, "UV-absorbing" refers to a material or compound, e.g. a polymeric or non-polymeric sunscreen agent or a chemical moiety, which absorbs radiation in some portion of the ultraviolet spectrum (290nm-400nm), such as one having an extinction coefficient of at least about 1000 $mol^{-1}$ $cm^{-1}$, for at least one wavelength within the above-defined ultraviolet spectrum. SPF values disclosed and claimed herein are determined using the in-vitro method described herein below.

The term "comprising" encompasses "including" as well as "consisting and "consisting essentially of", e.g. a composition "comprising" X may consist exclusively of X or may include something additional e.g. X and Y.

### UV-ABSORBING POLYGLYCEROL

Embodiments of the invention relate to compositions including an ultraviolet radiation absorbing polyglycerol, (i.e., "UV-absorbing polyglycerol"). By UV-absorbing polyglycerol, it is meant a polyglycerol that absorbs radiation in some portion of the ultraviolet spectrum (wavelengths between 290 and 400 nm). The UV-absorbing polyglycerol has a weight average molecular weight ($M_w$) which may be suitable for reducing or preventing the chromophore from absorbing through the skin. According to one embodiment, a suitable molecular weight for the UV-absorbing polyglycerol is $M_w$ greater than 500. In one embodiment, $M_w$ is in the range of about 500 to about 50,000. In another embodiment, $M_w$ is in the range of about 500 to about 5,000. In another embodiment, the $M_w$ is in the range of about 1,000 to about 20,000,

such as from about 1,000 to about 10,000.

Described herein is a composition including a UV-absorbing polyglycerol. As one skilled in the art will recognize, "polyglycerol" indicates that the UV-absorbing polymer includes a plurality of glyceryl repeat units covalently bonded to each other. The "backbone" of the UV-absorbing polyglycerol refers to the longest continuous sequence of covalently bonded glyceryl repeat units. Other smaller groups of covalently bonded atoms are considered pendant groups that branch from the backbone.

By "glyceryl repeat units" (also referred to herein as "glyceryl remnant units") it is meant glycerol units excluding nucleophilic groups such as hydroxyl groups. Glyceryl remnant units include ether functional groups, and generally may be represented as $C_3H_5O$ for linear and dendritic remnants (Rokicki et al. Green Chemistry., 2005, 7, 52). Suitable glyceryl remnant units include dehydrated forms (i.e. one mole of water removed) of the following glyceryl units: linear-1,4 ($L_{1,4}$) glyceryl units; linear-1,3 ($L_{1,3}$) glyceryl repeat units; dendritic (D) glyceryl units; terminal-1,2 ($T_{1,2}$) units; and terminal-1,3 ($T_{1,3}$) units. Examples of linear glyceryl remnant units and terminal units are shown below (to the right side of the arrows). The corresponding glyceryl unit before dehydration (shown to the left side of arrows; includes hydroxyls) are shown as well:

linear-1,4 ($L_{1,4}$) glyceryl repeat units

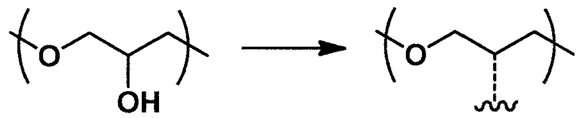

linear 1,4 linear 1,4 remnant

linear-1,3 ($L_{1,3}$) glyceryl repeat units

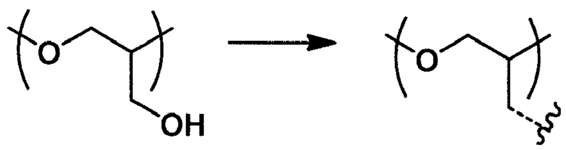

linear 1,3 linear 1,3 remnant

terminal-1,2 ($T_{1,2}$) units

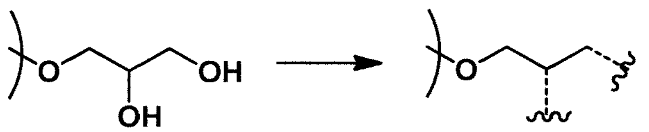

terminal 1,2 terminal 1,2 remnant

and terminal-1,3 ($T_{1,3}$) units

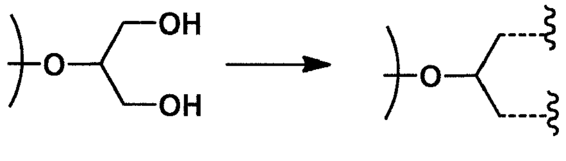

terminal 1,3 terminal 1,3 remnant

Those skilled in the art of polymer chemistry will recognize that a polyglycerol, like any typical polymer, is comprised of repeating units and end groups. In the simple case of a polymer formed by condensation of monomer units (elimination of water during polymerization), the end groups are comprised of the parent molecule, while the repeating unit is derived from the parent monomer minus a water molecule. Such is the case for polyglycerols, which can be

synthesized by using the monomer glycerol.

This is further illustrated in the Structure I below, where repeating unit isomers have been demarcated by parentheses (7 total glyceryl repeat units) and the terminal glyceryl remnant demarcated by brackets (1 terminal glyceryl remnant), yielding a total degree of polymerization (DP) of 8. The "Z" in Structure I is selected from a UV-chromophore, a hydrophobic moiety, or an unreacted hydroxyl group.

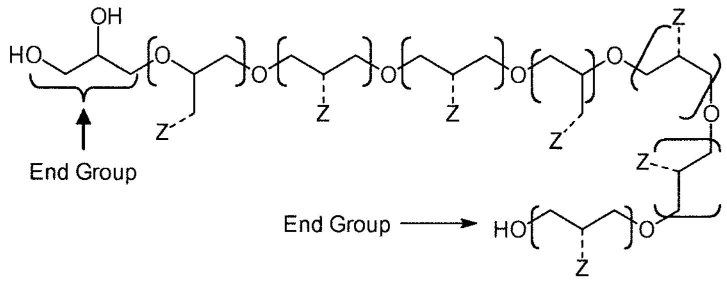


FORMULA I. UV-ABSORBING POLYGLYCEROL

As described above, the polyglycerol may include one or more hydrophobic moieties. Suitable hydrophobic moieties include, for example, nonpolar moieties that contain at least one of the following: (a) a carbon-carbon chain of at least six carbons in which none of the six carbons is a carbonyl carbon or has a hydrophilic moiety bonded directly to it; (b) three or more alkyl siloxy groups (-[Si(R)$_2$-O]-); and/or (c) three or more oxypropylene groups in sequence. A hydrophobic moiety may be, or include, linear, cyclic, aromatic, saturated or unsaturated groups. Preferred hydrophobic moieties include 6 or more carbon atoms, more preferably from 8 to 30 carbon atoms, even more preferably from 10 to 26 carbon atoms, and most preferably from 12 to 24 carbon atoms. Examples of hydrophobic moieties include linear or branched, saturated or unsaturated alkyl moieties, e.g. linear or branched, saturated or unsaturated $C_8$-$C_{30}$ alkyl, such as decyl, undecyl, dodecyl (lauryl), tridecyl, tetradecyl (myristyl), pentadecyl, hexadecyl (cetyl, palmityl), heptadecyl, heptadecenyl, hepta-8-decenyl, hepta-8,11-decenyl, octadecyl (stearyl), nonadecyl, eicosanyl, henicosen-12-yl, henicosanyl, docosanyl (behenyl), and the like as well as benzyl. Certain preferred hydrophobic moieties include heptadecyl, heptadecenyl, hepta-8-decenyl, hepta-8,11-decenyl and the like. Other examples of hydrophobic moieties include groups such as poly(oxypropylene), poly(oxybutylene), poly(dimethylsiloxane), and fluorinated hydrocarbon groups containing a carbon chain of at least six carbons in which none of the six carbons has a hydrophilic moiety bonded directly to it, and the like.

According to certain embodiments, polymer compositions of the present invention include low fractions of diglycerol chromophore conjugates. By "diglycerol chromophore conjugates," it is meant polyglycerols having two glyceryl repeat units and at least one chemically-bound UV-chromophore. An example structure is shown below in FORMULA II

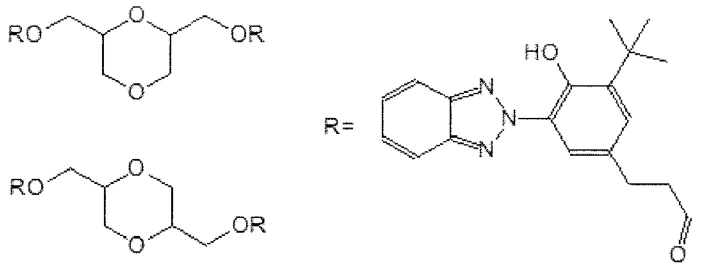

FORMULA II

By "low fractions" of diglycerol chromophore conjugates, it is meant that that the valley-to-valley peak area of peaks assignable to diglycerol chromophore conjugates in the ultraviolet radiation absorbing polyglycerol comprises about 1 percent or less of the total valley-to-valley peak area of all peaks in the spectrogram, i.e. "total peak area", such as about 0.5 percent or less of the total peak area, such as about 0.2 percent or less of the total peak area, as determined by chromatogram analysis, as set forth in the Examples below.

As one skilled in the art will readily recognize, one particularly suitable method for generating a chromatogram for determining the relative presence of chemical structures in a polymer composition involves the use of high performance

liquid chromatography (HPLC), ultraviolet/visible (UV-VIS) and mass spectrometry (MS). For example, the polymer composition may be tested for component analysis by separating components using HPLC. Detection is performed using ultraviolet/visible (UV-VIS) and mass spectrometry (MS) to generate a chromatogram of peaks at particular retention times, which peaks are assigned to individual components. According to certain embodiments of the invention, such analysis reveals no peak assignable to diglycerol chromophore conjugates. According to certain other embodiments, if such a peak exists, As one skilled in the art will readily appreciate, total peak area can be ascertained by connecting adjacent minima of points on the chromatogram and calculating (integrating) area under the curve for each of the various peaks. A specific suitable HPLC method for assessing the presence of diglycerol conjugates is provided below.

Polyglycerols described herein can be obtained through various synthetic routes. One particularly suitable route includes preparing a polyglycerol intermediate by polymerizing glycerol, such as by combining glycerol and suitable reactant such as an inorganic base (alkali) into a reactor and applying vacuum, agitation and heat in order to facilitate the polymerization of glycerol. By utilizing this method, glycerol is polymerized in a controlled fashion which tends to produce high polyglycerol intermediates with high linearity (less cyclic). According to one particular embodiment, the reactant is a multivalent inorganic base, such as a calcium-containing compound, such as calcium hydroxide. The temperature of the reactor may be maintained, for example between 200°C and 240°C, such as 220°C and 240°C. Suitable pressures may be from about 10mm Hg to about 400mm Hg, such as from about 100mm Hg to about 400mm Hg, such as about 150 mm Hg. Suitable molar ratios of glycerol to calcium-containing compound range from about 1:0.0002 to about 1:0.005.

According to certain embodiments, the polyglycerol intermediate is reacted with a hydrophobic reactant. Suitable hydrophobic reactants are those that are capable of displacing hydroxyl groups on the polyglycerol intermediate and covalently bonding thereto in order to ultimately provide a hydrophobic moiety (described above) bound to the UV-absorbing polyglycerol. As one skilled in the art will readily appreciate, suitable examples of hydrophobic reactants include linear or branched, saturated or unsaturated $C_8$-$C_{30}$ fatty acids, capable of reacting with hydroxyls on the polyglycerol intermediate and attaching to the polyglycerol via an ester linkage, $C_8$-$C_{30}$ isocyanates capable of reacting with hydroxyls via a urethane linkage. Other suitable hydrophobic reactants include $C_8$-$C_{30}$ epoxides, $C_8$-$C_{30}$ halohydrins, $C_8$-$C_{30}$ alkyl halides, among other hydrophobic reactants capable of condensation reactions with pendant hydroxyls on the polgylcerol intermediate. In this embodiment, a hydrophobically-modified polyglycerol intermediate is formed.

According to certain embodiments, the polyglycerol intermediate (or hydrophobically-modified polyglycerol intermediate) is enriched, i.e. residual glycerol and low molecular weight (low DP) fractions of polyglycerol are removed from the polyglycerol intermediate to form an enriched polyglycerol intermediate. Residual glycerol may be removed, for example, by heating and applying a vacuum. Suitable conditions for removing unreacted glycerol may be a temperature of about 200°C and pressure of about 4mm Hg. Additional glycerol may be removed by introducing steam through the bottom of the reactor

One particularly suitable method of removing glycerol as well as low DP components such as diglycerol includes applying heat and vacuum to the polyglycerol intermediate while the polyglycerol intermediate is drawn into a thin film. This so-called "wiped film evaporation" includes providing the polyglycerol intermediate to a chamber having a heated surface, applying vacuum, spreading thin films of the polyglycerol intermediate across the heated surface to selectively evaporate low molecular weight fractions of the polyglycerol intermediate. Spreading of the polyglycerol intermediate may be performed mechanically, such as via flexible blades that rotate about an axis and within the chamber, drawing the fluid polyglycerol intermediate into a film and facilitating evaporation and removal of fractions that are desirably removed, to form an enriched polyglycerol intermediate. Temperatures may be held at about 260°C and pressures at about 10 to 50 millitorr.

Suitable UV-chromophores that may be chemically bound in UV-absorbing polyglycerols of the present invention include UV-absorbing triazoles (a moiety containing a five-membered heterocyclic ring with two carbon and three nitrogen atoms), such as benzotriazoles. In another embodiment, the UV-absorbing chromophore of Formulas I and II includes a pendant UV-absorbing triazine (a six membered heterocycle containing three nitrogen and three carbon atoms). Suitable UV-chromophores include those that have absorbance of UVA radiation. Other suitable UV-chromophores are those which have absorbance in the UVB region. In one embodiment, the UV-chromophore absorbs in both the UVA and UVB region. In one embodiment, when the UV-absorbing polyglycerol is cast into a film, it is possible to generate a molar extinction coefficient measured for at least one wavelength in this wavelength range of at least about 1000 $mol^{-1}$ $cm^{-1}$, preferably at least about 2000 $mol^{-1}$ $cm^{-1}$, more preferably at least about 4000 $mol^{-1}$ $cm^{-1}$. In one embodiment, the molar extinction coefficient among at least 40% of the wavelengths in this portion of the spectrum is at least about 1000 $mol^{-1}$ $cm^{-1}$. Examples of UV-chromophores that are UVA absorbing include triazoles such as benzotriazoles, such as hydroxyphenyl-benzotriazoles; camphors such as benzylidene camphor and its derivatives (such as terephthalylidene dicamphor sulfonic acid); dibenzoylmethanes and their derivatives.

In one embodiment, the UV-chromophore is a benzotriazole providing both photostability and strong UVA absorbance with a structure represented in FORMULA III.

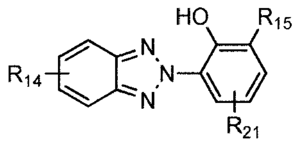

FORMULA III. BENZOTRIAZOLE UV-ABSORBING CHROMOPHORE wherein each $R_{14}$ is independently selected from the group consisting of hydrogen, $C_1$-$C_{20}$ alkyl, alkoxy, acyl, alkyloxy, alkylamino, and halogen; $R_{15}$ is independently selected from the group consisting of hydrogen, $C_1$-$C_{20}$ alkyl, alkoxy, acyl, alkyloxy, and alkylamino, $R_{21}$ is selected from $C_1$-$C_{20}$ alkyl, alkoxy, acyl, alkyloxy, and alkylamino. Either of the $R_{15}$ or $R_{21}$ groups may include the remnants of functional groups after reaction between the UV-chromophore and the enriched polyglycerol intermediate. Compounds resembling the structure in FORMULA III are described in U.S. Pat. No. 5,869,030, and include, but are not limited to, methylene bis-benzotriazolyl tetramethylbutylphenol (a compound sold under the trade name TINSORB M by BASF Corporation, Wyandotte, Michigan). In one embodiment, the UV-absorbing triazole is derived from a transesterification product of 3-(3-(2H-benzo[d][1,2,3]triazol-2-yl)-5-(tert-butyl)-4-hydroxyphenyl) propanoic acid with polyethylene glycol 300, commercially available as TINUVIN 213, also available from BASF. In another embodiment, the UV-absorbing triazole is Benzenepropanoic acid, 3-(2H-benzotriazol-2-yl)-5-(1,1-dimethylethyl)-4-hydroxy-, $C_{7-9}$-branched and linear alkyl esters, commercially available as TINUVIN 99, also available from BASF. In another embodiment, the UV-absorbing group contains a triazine moiety. An exemplary triazine is 6-octyl-2-(4-(4,6-di([1,1'-biphenyl]-4-yl)-1,3,5-triazin-2-yl)-3-hydroxyphenoxy) propanoate (a compound sold under the trade name TINUVIN 479 by BASF Corporation, Wyandotte, Michigan).

In another embodiment, the UV-chromophore is a UVB-absorbing moiety. By UVB-absorbing chromophore it is meant that the UV-chromophore has absorbance in the UVB portion (290 to 320 nm) of the ultraviolet spectrum. In one embodiment, the criteria for consideration as a UVB-absorbing chromophore is similar to those described above for an UVA-absorbing chromophore, except that the wavelength range is 290 nm to 320 nm. Examples of suitable UVB-absorbing chromophores include 4-aminobenzoic acid and alkane esters thereof; anthranilic acid and alkane esters thereof; salicylic acid and alkane esters thereof; hydroxycinnamic acid alkane esters thereof; dihydroxy-, dicarboxy-, and hydroxycarboxybenzophenones and alkane ester or acid halide derivatives thereof; dihydroxy-, dicarboxy-, and hydroxycarboxychalcones and alkane ester or acid halide derivatives thereof; dihydroxy-, dicarboxy-, and hydroxycarboxycoumarins and alkane ester or acid halide derivatives thereof; benzalmalonate (benzylidene malonate); benzimidazole derivatives (such as phenyl benzilimazole sulfonic acid, PBSA), benzoxazole derivatives, and other suitably functionalized species capable of being covalently bonded within the polymer chain. In another embodiment, the UV-absorbing polyglycerol includes more than one UV-chromophore, or more than one chemical class of UV-chromophore.

According to certain embodiments of the invention, in order to provide a chemically bound UV-chromophore on UV-absorbing polyglycerols present in polymer compositions of the present invention, a "post-polymerization attachment" technique may be employed. Unreacted, pendant hydroxyl groups present in the polyglycerol intermediate are reacted with a UV-chromophore containing a complementary functional group to obtain a UV-absorbing polyglycerol. Suitable complementary functional groups on UV-chromophore include carboxylates, isocyanates, epoxides, esters, alkyl esters, acid halides, and the like. One example of a UV-chromophore having complementary functional groups is a benzotriazole carboxylate UV-chromophore, 3-(3-(2H-benzo[d][1,2,3]triazol-2-yl)-5-(tert-butyl)-4-hydroxyphenyl) propanoic acid, shown below in FORMULA IV.

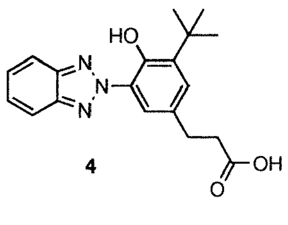

4

FORMULA IV

In this embodiment, the pendant hydroxyls on the enriched polyglycerol intermediate react via condensation with the complementary carboxylate functional group on the UV-chromophore. Benzotriazoles having carboxylate or other complementary functional groups may be prepared using methods known to those skilled in the art, such as those described in published U.S. patent application 2012/0058974, "Composition Comprising Pesticide and Benzotriazole UV Absorbers," which is herein incorporated by reference in its entirety.

Polymer compositions formed via these methods are accordingly the reaction product of enriched polyglycerol

intermediate or, alternatively, a hydrophobically modified polyglyerol, such as a polyglycerol ester, and a UV-chromophore having a functional group suitable for covalent attachment to the polyglycerol intermediate.

According to one specific embodiment, a polyglycerol intermediate is formed by polymerizing glycerol by reacting glycerol with calcium hydroxide. The temperature of the reactor is maintained between 200°C and 240°C, and pressure is maintained at about 400mm Hg and using a molar ratio of glycerol to calcium hydroxide from about 1:0.0002 to about 1:0.005. Residual glycerol and low molecular weight polyglycerols are removed using a wiped film evaporator having a barrel temperature of about 260°C and pressures at about 10 to 50 millitorr, thereby forming an enriched polyglycerol intermediate. The enriched polyglycerol intermediate is optionally esterified with stearic acid at elevated temperature (about 250°C) for several hours until clear, to form a hydrophobically-modified polyglycerol intermediate. Excess hydroxide is neutralized with phosphoric acid.

A benzotriazole carboxylate is prepared by adding, for example, the polyethylene glycol ester of 3-[3-(2H-1,2,3-benzotriazol-2-yl)-5-*tert*-butyl-4-hydroxyphenyl]propanoate (a chromophore sold under the trade name TINUVIN 213 by BASF Corporation, Wyandotte, Michigan). 81.0 g is added to a 2 L round bottom flask containing a magnetic stir bar. Ethanol (600 mL) is added to the flask by funnel, and the mixture is stirred until homogeneous. Sodium hydroxide (NaOH, 30.8 g) is dissolved in $H_2O$ (400 mL); the basic solution is transferred into an addition funnel above the 2L flask. The NaOH solution is added slowly to the stirred mixture. When addition is complete, the mixture is stirred overnight at room temperature. The solution is concentrated by rotary evaporation to remove most of the ethanol. The resulting orange oil is diluted to 1400 mL with $H_2O$. The mixture is stirred mechanically and acidified to ~pH 1 by addition of 1 M aq. HCl (~700 mL). The resulting white precipitate is filtered and pressed to remove water, then recrystallized from ethanol. The supernatant is removed and concentrated by rotary evaporation; a second crop of material is isolated as a white, amorphous solid. The two crops are combined and dried in a vacuum oven overnight to form a benzotriazole carboxylate.

The enriched, hydrophobically-modified polyglycerol is reacted with the benzotriazole carboxylate (8.8 g, 23.8 mmol) by transferring into a 2-neck 100 mL round bottom flask containing a magnetic stir bar. The flask is fitted with a nitrogen inlet adapter and distillation adapter with 100 mL receiving flask. The apparatus is placed under vacuum for one hour, then backfilled with nitrogen. The distillation head is removed, and tin (II) ethyl hexanoate (50 $\mu$L) is added to the reaction flask by syringe under nitrogen flow. The apparatus is reassembled, then purged under vacuum and backfilled with nitrogen 3 times. The reaction flask is immersed in an oil bath that was warmed to 180 °C with constant flow of nitrogen into the 2-neck flask through the distillation adapter and out of the vacuum adapter to room atmosphere. The reaction is stirred for three hours and then cooled to room temperature under nitrogen flow, affording the product, a polymer composition including UV-absorbing polyglycerol, as a yellow solid.

As one skilled in the art will recognize, the UV-absorbing polyglycerols that are useful in topical compositions of the present invention are prepared via polymer synthesis. Synthesis of the UV-absorbing polyglycerol generally results in a reaction product, hereinafter referred to as a "polymer composition", that is a mixture of various molecular weights of UV-absorbing polyglycerols. Despite the removal/reduction of glycerol and low-molecular glycerol conjugates, the polymer composition may further include (apart from the UV-absorbing polyglycerol composition) a small amount of unbound, i.e. unconjugated, material, e.g., glycerol, chromophore or hydrophobic moieties that are not covalently bound to the polyglycerol backbone.

According to certain embodiments, the polymer composition to be incorporated into topical compositions of the present invention comprises about 90% or more of the UV-absorbing polyglycerol that comprises a UV-chromophore chemically bound thereto. According to certain other embodiments, the polymer composition comprises about 95% or more of the UV-absorbing polyglycerol that comprises a UV-chromophore chemically bound thereto. According to certain other embodiments, the polymer composition comprises about 98% or more of the linear UV-absorbing polyglycerol having a chromophore chemically bound thereto, such as about 99% or more.

The polymer compositions described herein are useful in applications where UV absorption is desired. For example, the polymer composition may be useful for combining with a suitable cosmetically acceptable carrier for cosmetic applications or combining the polymer composition with other materials to reduce UV degradation of the materials (i.e., melt blending the material with the polymer composition or coating the material with the UV-absorbing polymer). The incorporation of UV-absorbing polyglycerols into such compositions of the present invention may provide enhanced SPF (primarily UVB absorbance), enhanced PFA (primarily UVA absorbance) or enhancement of both. The cosmetically-acceptable topical carrier is suitable for topical application to human skin and may include for example, one or more of vehicles such as water, ethanol, isopropanol, emollients, humectants, and/or one or more of surfactants/emulsifiers, fragrances, preservatives, water-proofing polymers, and similar ingredients commonly used in cosmetic formulations. As such, the polymer composition may be formulated using ingredients known in the art into a spray, lotion, gel, stick or other product forms. Similarly, according to certain embodiments, one may protect human skin from UV radiation by topically applying a composition comprising the polymer composition containing the UV-absorbing polyglycerol.

According to certain embodiments, the sunscreen agent present in topical compositions of the present invention may consist of, or consists essentially of, the UV-absorbing polyglycerol having the chromophore chemically bound

thereto, as defined herein. According to certain other embodiments, the sunscreen agent may include additional UV-absorbing polymers, other than those UV-absorbing polyglycerols, as defined herein, and/or non-UV-absorbing, light-scattering particles. Additional UV-absorbing polymers are molecules that can be represented as having one or more structural units that repeat periodically, e.g., at least twice, to generate the molecule, and may be UV-absorbing polyglycerols, other than those as defined and claimed in this specification. In certain embodiments, the compositions may be substantially free of UV-absorbing polymers other than the UV-absorbing polyglycerols. By "substantially free of UV-absorbing polymers other than the UV-absorbing polyglycerols", it is meant that the compositions do not contain UV-absorbing polymers other than the UV-absorbing polyglycerols in an amount effective to provide the compositions with an SPF of greater than 2 in the absence of the UV-absorbing polyglycerol and non-polymeric UV-absorbing sunscreen agents. In yet other embodiments, the compositions may be substantially free of both UV-absorbing polymers other than the UV-absorbing polyglycerols and non-polymeric UV-absorbing sunscreen agents, as described below. For example, the compositions of the invention will contain about 1% or less, or about 0.5% or less, of such UV-absorbing polymers other than the UV-absorbing polyglycerols and/or such non-polymeric absorbing UV-absorbing sunscreen agents.

Additional UV-absorbing polymers may have a molecular weight of greater than about 1500. Examples of suitable additional UV-absorbing polymers include benzylidene malonate silicone, including those described in US Patent 6,193,959, to Bernasconi et al. A particularly suitable benzylidene malonate includes "Parsol SLX," commercially available from DSM (Royal DSM N.V.) of Heerlen, Netherlands. Other suitable additional UV-absorbing polymers are disclosed in US 6, 962,692; US 6,899, 866; and/or US 6,800,274; including hexanedioic acid, polymer with 2,2-dimethyl-1,3-propanediol, 3-[(2-cyano-1-oxo-3,3-diphenyl-2-propenyl)oxy]-2,2-dimethylpropyl 2-octyldodecyl ester; sold under the trade name "POLYCRYLENE," commercially available from the HallStar Company of Chicago, Illinois. When utilized, such additional UV-absorbing polymers may be used at concentrations of about 1% or more, for example about 3% or more.

Non-UV-absorbing, light-scattering particles do not absorb in the UV spectrum, but may enhance SPF by scattering of the incident UV radiation. Examples of non-UV-absorbing, light-scattering particles include solid particles having a dimension, e.g., average diameter, from about 0.01 micron to about 10 microns. In certain embodiments, the non-UV-absorbing, light-scattering particle is a hollow particle comprising, or consisting essentially of, an organic polymer or a glass. Suitable organic polymers include acrylic polymers, including acrylic/styrene copolymers, such as those known as SUNSPHERES, which are commercially available from Dow Chemical of Midland, Michigan. Suitable glasses include borosilicate glasses such as those described in published United States Patent Application US20050036961A1, entitled, "AESTHETICALLY AND SPF IMPROVED UV-SUNSCREENS COMPRISING GLASS MICROSPHERES".

<u>TOPICAL COMPOSITION</u>

In one embodiment, a composition suitable for topical/cosmetic use for application to the human body, e.g., keratinaceous surfaces such as the skin, hair, lips, or nails, and especially the skin, is provided. The composition includes the polymer composition comprising the UV-absorbing polyglycerols that comprise a UV-chromophore chemically bound thereto.

As discussed above, the concentration of the UV-absorbing polyglycerol comprise a UV-chromophore chemically bound thereto in the topical composition may be sufficient to provide an SPF of about 10 or greater, particularly where the composition is free of, or substantially free of, additional UV-absorbing polymers, i.e. UV-absorbing polymers other than the UV-absorbing polyglycerols comprising a UV-chromophore chemically bound thereto, or non-polymeric UV-absorbing sunscreen agents as described herein. Accordingly, the concentration of the UV-absorbing polyglycerol may vary from about 5% to about 50%, such as from about 7% to about 40%, such as from about 10% to about 30%, such as from about 15% to about 30% of the composition. In certain embodiments, the concentration of UV-absorbing polyglycerol is about 10% or more, such as about 15% or more, such about 25% or more of the composition. According to certain embodiments where the sunscreen agent consists essentially of the UV-absorbing polyglycerol, the concentration of the UV-absorbing polyglycerol may be about 15% or more.

The concentration of non-UV-absorbing light scattering particles, if present, may be about 1% or more, such as from about 1% to about 10%, such as from about 2% to about 5%. In certain embodiments where the UV-sunscreen agent further includes a non-UV-absorbing sunscreen agent in amounts as discussed above, compositions of the present invention may have an SPF of about 20 or greater.

Compositions of the present invention, according to certain embodiments, may be substantially free of non-polymeric UV-absorbing sunscreen agents. By "substantially free of non-polymeric UV-absorbing sunscreen agents," it is meant that, in this embodiment, the compositions do not contain non-polymeric UV-absorbing sunscreen agents in an amount effective to provide the compositions with an SPF of greater than 2 in the absence of the UV-absorbing polyglycerol and UV-absorbing polymers other than the UV-absorbing polyglycerols used in the present invention, as determined via the in vitro method described herein below. For example, the compositions of the invention will contain about 1% or less, or about 0.5% or less, of such non-polymeric UV-absorbing sunscreen agents. One example of non-polymeric UV-absorbing sunscreen agents that the composition is substantially free of typically may be characterized as "organic" (include predominantly or only atoms selected from carbon, hydrogen, oxygen, and nitrogen) and having no definable repeat unit and typically having molecular weights that are about 600 daltons or less, such as about 500 daltons or less, such as less than 400 daltons. Examples of such compounds, sometimes referred to as "monomeric, organic UV-absorbers" include, but are not limited to: methoxycinnamate derivatives such as octyl methoxycinnamate and isoamyl methoxycinnamate; camphor derivatives such as 4-methyl benzylidene camphor, camphor benzalkonium methosulfate, and terephthalylidene dicamphor sulfonic acid; salicylate derivatives such as octyl salicylate, trolamine salicylate, and homosalate; sulfonic acid derivatives such as phenylbenzimidazole sulfonic acid; benzone derivatives such as dioxybenzone, sulisobenzone, and oxybenzone; benzoic acid derivatives such as aminobenzoic acid and octyldimethyl para-amino benzoic acid; octocrylene and other β,β-diphenylacrylates; dioctyl butamido triazone; octyl triazone; butyl methoxydibenzoyl methane; drometrizole trisiloxane; and menthyl anthranilate.

Other non-polymeric UV-absorbing sunscreen agents that the composition may be substantially free of may include ultraviolet-absorbing particles, such as certain inorganic oxides, including titanium dioxide, zinc oxide, and certain other transition metal oxides. Such ultraviolet screening particles are typically solid particles having a diameter from about 0.1 micron to about 10 microns.

The compositions of the present invention may be used for a variety of cosmetic uses, especially for protection of the skin from UV radiation. The compositions, thus, may be made into a wide variety of delivery forms. These forms include, but are not limited to, suspensions, dispersions, solutions, or coatings on water soluble or water-insoluble substrates (e.g., substrates such as organic or inorganic powders, fibers, or films). Suitable product forms include lotions, creams, gels, sticks, sprays, ointments, mousses, and compacts/powders. The composition may be employed for various end-uses, such as recreation or daily-use sunscreens, moisturizers, cosmetics/make-up, cleansers/toners, anti-aging products, or combinations thereof. The compositions of the present invention may be prepared using methodology that is well known by an artisan of ordinary skill in the field of cosmetics formulation.

<u>TOPICAL CARRIER</u>

The one or more UV-absorbing polymers in the composition may be combined with a "cosmetically-acceptable topical carrier," i.e., a carrier for topical use that is capable of having the other ingredients dispersed or dissolved therein, and possessing acceptable properties rendering it safe to use topically. As such, the composition may further include any of various functional ingredients known in the field of cosmetic chemistry, for example, emollients (including oils and waxes) as well as other ingredients commonly used in personal care compositions, such as humectants, thickeners, opacifiers, fragrances, dyes, solvents for the UV-absorbing polyglycerol, among other functional ingredients. Suitable examples of solvents for the UV-absorbing polyglycerol include dicaprylyl carbonate available as CETIOL CC from Cognis Corporation of Ambler, Pennsylvania. In order to provide pleasant aesthetics, in certain embodiments of the invention, the composition is essentially free of volatile solvents, and, in particular, $C_1$-$C_4$ alcohols such as ethanol and isopropanol.

Furthermore, the composition may be essentially free of ingredients that would render the composition unsuitable for topical use. As such, the composition may be essentially free of solvents such as volatile solvents, and, in particular, free of volatile organic solvents such as ketones, xylene, toluene, and the like.

Sun protection factor (SPF) may be tested using the following IN-VITRO SPF TEST METHOD. The baseline transmission of a PMMA plate (substrate) without application of any test materials applied thereto was measured. Test samples were prepared by providing a sample of polymer. Blends may also be tested by this method. The polymer(s) can be tested without any additional additives; with a solvent system, or as a part of a personal care composition that may include solvent and/or additional ingredients.

Each sample is separately applied to a PMMA plate (available from Helioscience, Marseille, France) using an application density of about 1.3 mg/cm2, rubbing into a uniform thin layer with the operator’s finger, and allowing to dry. The samples are allowed to dry for 15 minutes before measurement of absorbance using calibrated Labsphere® UV-10005 UV transmission analyzer or a Labsphere® UV-2000S UV transmission analyzer (Labsphere, North Sutton, N.H., USA). The absorbance measures are used to calculate SPF and PFA indices.

SPF and PFA may be calculated using methods known in the art - see equation (1) below for calculation of SPF:

$$\text{SPF}_{\text{in vitro}} = \frac{\int_{\lambda=290nm}^{\lambda=400nm} E(\lambda) * I(\lambda) * d\lambda}{\int_{\lambda=290nm}^{\lambda=400nm} E(\lambda) * I(\lambda) * 10^{-A_0(\lambda)} * d\lambda} \qquad (1)$$

where:

E(λ) = Erythema action spectrum
I(λ) = Spectral irradiance received from the UV source
A0(λ) = Mean monochromatic absorbance of the test product layer before UV exposure
dλ = Wavelength step (1 nm)

The compositions of the present invention may be prepared using mixing and blending methodology that is well known by an artisan of ordinary skill. In one embodiment of the invention, a method of making a composition of the present invention includes preparing an oil phase by mixing at least the UV-absorbing polyglycerol with optional oil-soluble or oil-miscible ingredients; and preparing a water phase, by mixing water and optional water-soluble or water-miscible ingredients. The oil phase and the water phase may then be mixed in a manner sufficient to homogeneously disperse the oil phase in the water phase such that the water phase is continuous and the oil phase discontinuous.

The compositions of the present invention can be used by topically administering to a mammal, e.g., by the direct laying on, wiping or spreading of the composition on the skin or hair of a human.

The following HPLC TEST is used in the instant methods and in the following Examples. In particular, as described above, the HPLC TEST METHOD is used to determine whether the polymer composition includes diglycerol chromophore conjugates.

**HPLC TEST:**

The HPLC (HIGH PERFORMANCE LIQUID CHROMATOGRAPHY) TEST is designed to determine the relative concentrations of various components in the polymer composition. The components of the polymer composition are separated by high performance liquid chromatography (HPLC) and detected using ultraviolet visible (UV) spectrometry and mass spectrometry (MS) to generate a chromatogram where each of the chromatographic peaks at specific retention time corresponds to an individual component within the polymer composition. Higher peak response (HPLC-UV peak area in this document) indicates a higher concentration of a particular component. The values of HPLC-UV peak area are obtained through a valley-to-valley integration using a well-qualified data process software (LC/MSD ChemStation, Rev. B.0403-SP1 (87), Agilent Technologies, Inc., 2850 Centerville Road, Wilmington, DE 19808-1610, USA). The molecular weight of each of the components is obtained using mass spectrometric detection. Mass spectrometry directly measures the molecular weight of each of the components after ionization. The mass spectrometer is calibrated using an Agilent ESI Tuning Mix (Part # G2421A) standard with known molecular weight to ensure the accuracy of the instrumentation. The molecular weight information is used to propose corresponding chemical structures.

As one skilled in the art will readily recognize, the presence of diglycerol chromophore conjugates in the polymer composition can be determined by calculating the molecular weight of the diglycerol conjugate from its chemical structure, and matching the molecular weight with retention time. For example, for a polymer compostition that is formed by reacting an enriched polyglycerol ester intermiedate with the benzotriazole carboxylate UV-chromophore, 3-(3-(2H-benzo[d][1,2,3]triazol-2-yl)-5-(tert-butyl)-4-hydroxyphenyl) propanoic acid, the diglycerol conjugate shown in Formula II has a molecular weight of 790.4.

The following examples are illustrative of the principles and practice of this invention, although not limited thereto. Numerous additional embodiments within the scope and spirit of the invention will become apparent to those skilled in the art once having the benefit of this disclosure.

**EXAMPLES**

EXAMPLE I

Two polymer compositions comprising a UV-absorbing polyglycerol were analyzed using the HPLC METHOD. Inventive Polymer Composition A was made using a controlled process designed to provide low fractions of diglycerol

chromophore conjugates, while Comparative Polymer Composition B was made using a conventional process. For each of the polymer compositions, 100 mg of polymer composition was dissolved in 20 ml of tetrahydrofuran (THF) to a concentration of 5,000 ppm followed with 5 times dilution with THF to a final concentration of 1,000 ppm. The solutions were analyzed according to the HPLC TEST using an Agilent LC/MSD SL, ID# SK 1519 (Agilent technologies, Santa Clara, California). An Agilent Zorbax Eclipse XDBC8 column (3.5 micron, 150x3mm ID, S/N: USOX002084) was employed with mobile phase A (20mM ammonium acetate in water: ACN (10:90) and mobile phase B (isopropanol: ethyl acetate: 100mM ammonium acetate (50:50:2). Elution time, flow rate, and relative amounts of mobile phases are provided in Table 1, below:

TABLE 1: HPLC TEST: MOBILE PHASES

| Binary gradient elution: Time (min) | Flow rate (mL/min) | Mobile phase A (%) | Mobile phase B (%) |
|---|---|---|---|
| 0.00 | 0.30 | 100.0 | 0.0 |
| 5.00 | 0.30 | 90.0 | 10.0 |
| 40.00 | 0.30 | 60.0 | 40.0 |
| 80.00 | 0.30 | 30.0 | 70.0 |
| 90.00 | 0.30 | 5.0 | 95.0 |
| 95.00 | 0.30 | 5.0 | 95.0 |
| 100.00 | 0.30 | 100.0 | 0.0 |
| 110.00 | 0.30 | 100.0 | 0.0 |

UV detection was set at 305nm. Mass spectrometric detection was set for electrospray Ionization (ESI), positive ion mode with a scan setting of 650-3,000 amu; fragmentor: 400; fain: 1.0; and drying gas temp at 350°C.

The HPLC-UV peaks and their retention times (RT) are summarized in Table 2. Two relative major peaks, peak #2 (RT 8.88 min) and peak #4 (RT 12.45 min), were observed in Polymer Composition B, but not observed in Polymer Composition A.

The species observed from HPLC-UV (Figure 1) were analyzed with a mass spectrometric detector as indicated. The results were summarized in Table 3.

Mass spectrometric analysis indicates that Polymer Composition A has no peaks assignable to the lowest molecular weight glycerol conjugates, specifically diglycerol conjugates (i.e., structures recorded as "(G2-H2O)_T2") correlating to retention times (RT) 8.88 and 12.45 minutes. In contrast, Polymer Composition B has 4.93% (RT 8.88) and 5.81% (RT 12.45), respectively, for a combined peak area of 10.74% for the respective retention times (RT 8.88 and RT 12.45). This indicates a surprising reduction in the percent of diglycerol conjugates present in Polymer Composition A, as compared with Polymer Composition B.

TABLE 2: HPLC TEST: UV ANALYSIS

| PEAK NUMBER | Retention Time (min) | % OF TOTAL PEAK AREA Polymer Composition A | % OF TOTAL PEAK AREA Polymer Composition B |
|---|---|---|---|
| 1 | 6.72 | NA | 0.54 |
| 2 | 8.88 | 4.93 | NA |
| 3 | 9.56 | NA | 0.78 |
| 4 | 12.45 | 5.81 | NA |
| 5 | 13.96 | NA | 2.20 |
| 6 | 18.02 | NA | 1.94 |
| 7 | 19.75 | NA | 0.72 |
| 8 | 21.06 | 3.76 | 2.81 |
| 9 | 23.60 | 1.07 | 4.37 |
| 10 | 26.19 | 3.73 | 2.48 |
| 11 | 28.52 | 1.66 | 2.65 |

(continued)

| PEAK NUMBER | Retention Time (min) | % OF TOTAL PEAK AREA Polymer Composition A | % OF TOTAL PEAK AREA Polymer Composition B |
|---|---|---|---|
| 12 | 31.71 | NA | 6.24 |
| 13 | 34.15 | 1.75 | 1.31 |
| 14 | 36.64 | 4.68 | 14.84 |
| 15 | 38.75 | 2.34 | 2.24 |
| 16 | 40.91 | 5.65 | 5.34 |
| 17 | 42.67 | 0.70 | 4.05 |
| 18 | 43.91 | 1.13 | 2.11 |
| 19 | 46.12 | 5.07 | 7.87 |
| 20 | 46.83 | NA | 6.36 |
| 21 | 47.83 | 1.51 | NA |
| 22 | 49.93 | 6.82 | 9.17 |
| 23 | 51.38 | 1.54 | NA |
| 24 | 53.32 | 4.16 | 1.67 |
| 25 | 54.63 | 2.95 | 2.94 |
| 26 | 56.76 | 5.87 | 4.39 |
| 27 | 58.98 | 5.42 | 2.40 |
| 28 | 60.06 | NA | 0.54 |
| 29 | 61.39 | 2.95 | 3.23 |
| 30 | 62.23 | 3.79 | NA |
| 31 | 64.26 | 4.73 | 2.55 |
| 32 | 66.68 | 5.07 | 1.95 |
| 33 | 69.02 | 2.91 | 1.36 |
| 34 | 70.65 | 3.66 | 0.96 |
| 35 | 72.48 | 0.65 | NA |
| 36 | 73.93 | 2.07 | NA |
| 37 | 75.19 | 0.57 | NA |
| 38 | 76.61 | 1.38 | NA |
| 39 | 78.71 | 0.67 | NA |

TABLE 3: HPLC TEST: MASS SPECTROMETRY (MS) ANALYSIS

| **Peak #** | **RT (min)** | **MW** | **Nominal Ions (M+Na+)** | **Nominal Ions (M+2Na+)** | **Observed Ions (M+Na+)** | **Observed Ions (M+2Na+)** | **Proposed Structure** |
|---|---|---|---|---|---|---|---|
| 1 | 6.72 | 864.4 | 887.4 | | 887.3 | | (G3-H2O) T2 |
| 2 | 8.88 | 790.4 | 813.4 | | 813.2 | | (G2-H2O) T2 |
| 3 | 9.56 | 864.4 | 887.4 | | 887.3 | | (G3-H2O) T2 |
| 4 | 12.45 | 790.4 | 813.4 | | 813.2 | | (G2-H2O) T2 |

(continued)

| Peak # | RT (min) | MW | Nominal Ions (M+Na+) | Nominal Ions (M+2Na+) | Observed Ions (M+Na+) | Observed Ions (M+2Na+) | Proposed Structure |
|---|---|---|---|---|---|---|---|
| 5 | 13.96 | 1204.7 | 1227.7 | | 1227.8 | | C18-(G4-H2O) T2 |
| 6 | 18.02 | 1130.7 | 1153.7 | | 1153.3 | | C18-(G3-H2O) T2 |
| 7 | 19.75 | 1130.7 | 1153.7 | | 1153.3 | | C18-(G3-H2O) T2 |
| 8 | 21.06 | 1186.7 | 1209.7 | | 1209.3 | | C18-(G4-2H2O) T2 |
| 9 | 23.60 | 1130.7 | 1153.7 | | 1153.3 | | C18-(G3-H2O) T2 |
| 10 | 26.19 | 1186.7 | 1209.7 | | 1209.3 | | C18-(G4-2H2O) T2 |
| 11 | 28.52 | 1599.9 | 1622.9 | | 1622.4 | | C18-(G5-H2O) T3 |
| 12 | 31.71 | 1525.9 | 1548.9 | | 1548.4 | | C18-(G4-H2O) T3 |
| 13 | 34.15 | 1599.9 | 1622.9 | | 1622.4 | | C18-(G5-H2O) T3 |
| 14 | 36.64 | 1525.9 | 1548.9 | | 1548.4 | | C18-(G4-H2O) T3 |
| 15 | 38.75 | 1581.9 | 1604.9 | | 1604.4 | | C18-(G5-2H2O) T3 |
| 16 | 40.91 | 1451.8 | 1474.8 | | 1474.4 | | C18-(G3-H2O) T3 |
| 17 | 42.67 | 1921.0 | 1944.0 | | 1943.6 | | C18-(G5-H2O) T4 |
| 18 | 43.91 | 1995.1 | 2018.1 | | 2017.6 | | C18-(G6-H2O) T4 |
| 19 | 46.12 | 1847.0 | 1870.0 | | 1869.5 | | C18-(G4-H2O) T4 |
| 20 | 46.83 | 1921.0 | 1944.0 | | 1943.5 | | C18-(G5-H2O) T4 |
| 21 | 47.83 | 1977.1 | 2000.1 | | 1999.6 | | C18-(G6-2H2O) T4 |
| 22 | 49.93 | 1847.0 | 1870.0 | | 1869.5 | | C18-(G4-H2O) T4 |
| 23 | 51.38 | 2372.2 | 2395.2 | | 2394.6 | | C18-(G7-2H2O)_T5 |
| 24 | 53.32 | 2242.2 | 2265.2 | | 2264.6 | | C18-(G5-H2O)_T5 |
| 25 | 54.63 | 2316.2 | 2339.2 | | 2338.6 | | C18-(G6-H2O) T5 |
| 26 | 56.76 | 2242.2 | 2265.2 | | 2264.6 | | C18-(G5-H2O)_T5 |
| 27 | 58.98 | 2637.4 | 2660.4 | | 2659.8 | | C18-(G6-H2O)_T6 |
| 28 | 60.06 | 2711.4 | | 1378.7 | | 1378.8 | C18-(G7-H2O)_T6 |
| 29 | 61.39 | 2767.4 | | 1406.7 | | 1406.6 | C18-(G8-2H2O)_T6 |
| 30 | 62.23 | 3162.6 | | 1604.3 | | 1603.9 | C18-(G9-2H2O) T7 |
| 31 | 64.26 | 3032.5 | | 1539.3 | | 1538.9 | C18-(G7-H2O)_T7 |
| 32 | 66.68 | NA | | | | 2209.8 | NA |
| 33 | 69.02 | 2711.4 | | 1378.7 | | 1378.8 | C18-(G7-H2O) T6 |
| 34 | 70.65 | 3427.7 | | 1736.9 | | 1736.4 | C18-(G8-H2O) T8 |
| 35 | 72.48 | 3106.6 | | 1576.3 | | 1576.4 | C18-(G8-H2O) T7 |
| 36 | 73.93 | 3822.9 | | 1934.5 | | 1933.9 | C18-(G9-H2O) T9 |
| 37 | 75.19 | NA | | | | | NA |
| 38 | 76.61 | NA | | | | | NA |
| 39 | 78.71 | NA | | | | | NA |
| 40 | 79.54 | NA | | | | | NA |

It is understood that while the invention has been described in conjunction with the detailed description thereof, that the foregoing description is intended to illustrate and not limit the scope of the invention.

## Claims

1. A polymer composition comprising an ultraviolet radiation absorbing polyglycerol comprising a UV-chromophore chemically bound thereto, wherein a valley-to-valley peak area of chromatogram peaks assignable to diglycerol chromophore conjugates in said ultraviolet radiation absorbing polyglycerol comprises about 1 percent or less of a total valley-to-valley peak area of all chromatogram peaks.

2. The polymer composition of claim 1, wherein said valley-to-valley peak area of said chromatogram peaks assignable to said diglycerol chromophore conjugates is about 0.5 percent or less of said total peak area of all of said chromatogram peaks.

3. The polymer composition of claim 1, wherein said valley-to-valley peak area of said chromatogram peaks assignable to said diglycerol chromophore conjugates is about 0.2 percent or less of said total peak area of all of said chromatogram peaks.

4. A method of forming a polymer composition comprising an ultraviolet radiation absorbing polyglycerol, the method comprising:

   preparing a polyglycerol intermediate by reacting glycerol with a multivalent inorganic base; and
   reacting said polyglycerol intermediate with a UV-chromophore having a complementary functional group to form said polymer composition.

5. The method of claim 4, further comprising removing residual glycerol and low molecular weight fractions from said polyglycerol intermediate to form an enriched polyglycerol intermiedate, and reacting said enriched polyglycerol intermiedate with said UV-chromophore having a complementary functional group to form said polymer composition.

6. A composition comprising:

   a polymer composition according to claim 1 comprising an ultraviolet radiation absorbing polyglycerol comprising a UV-chromophore chemically bound thereto, wherein a valley-to-valley peak area of chromatogram peaks assignable to diglycerol chromophore conjugates in said ultraviolet radiation absorbing polyglycerol comprises about 1 percent or less of a total valley-to-valley peak area of all chromatogram peaks; and
   a cosmetically-acceptable topical carrier.

FIGURE 1

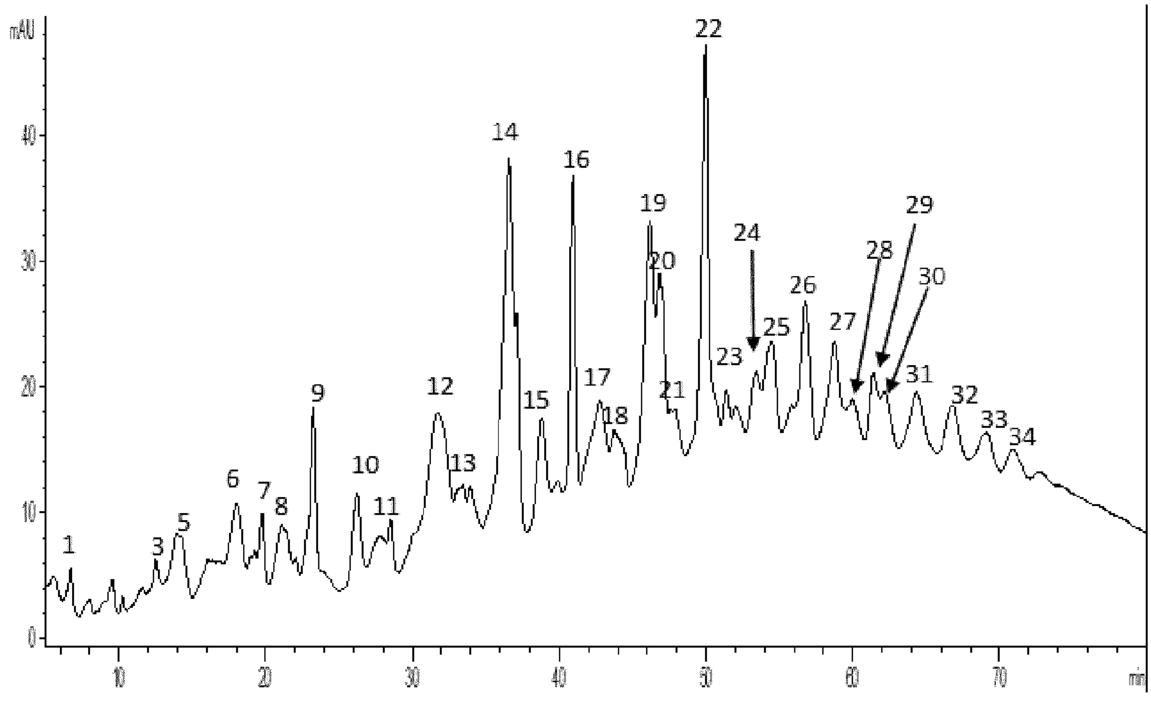
mAU
40
30
20
10
0
10
20
30
40
50
60
70
min
1
3
5
6
7
8
9
10
11
12
13
14
15
16
17
18
19
20
21
22
23
24
25
26
27
28
29
30
31
32
33
34

FIGURE 2

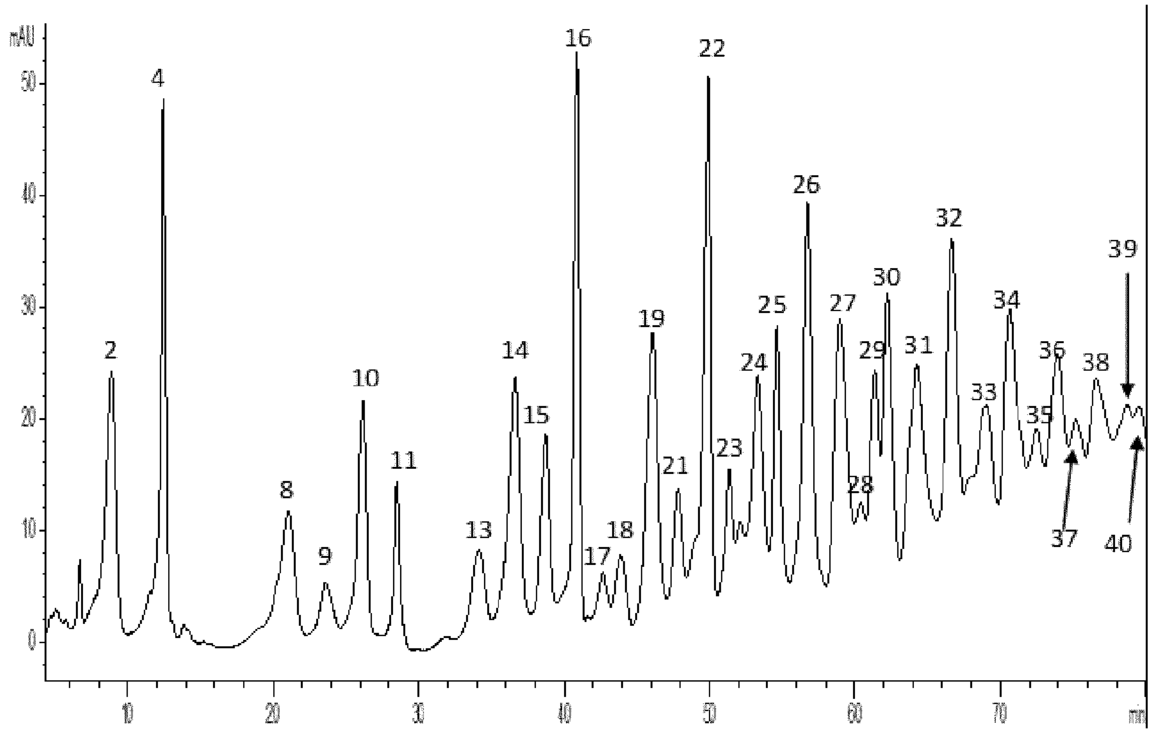
mAU
50
40
30
20
10
0
10
20
30
40
50
60
70
min
2
4
8
9
10
11
13
14
15
16
17
18
19
21
22
23
24
25
26
27
28
29
30
31
32
33
34
35
36
37
38
39
40

Europäisches Patentamt
European Patent Office
Office européen des brevets

# EUROPEAN SEARCH REPORT

Application Number
EP 14 19 8419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P<br><br>Y,P | EP 2 679 616 A1 (JOHNSON & JOHNSON CONSUMER [US]) 1 January 2014 (2014-01-01)<br>* claims 1-15 *<br>* examples 2,11 * | 1-3,6<br><br>4,5 | INV.<br>A61K8/86<br>A61Q17/04 |
| X,P<br><br>Y,P | WO 2014/004474 A2 (JOHNSON & JOHNSON CONSUMER [US]) 3 January 2014 (2014-01-03)<br>* claims 1-29 *<br>* examples 2,11 * | 1-3,6<br><br>4,5 | |
| X<br><br><br>Y | WO 2011/070050 A1 (DSM IP ASSETS BV [NL]; SCHLIFKE-POSCHALKO ALEXANDER [CH])<br>16 June 2011 (2011-06-16)<br>* comparative example page 21 * | 1-3,6<br><br><br>4,5 | |
| X<br><br><br>Y | WO 2005/092282 A1 (DSM IP ASSETS BV [NL]; POSCHALKO ALEXANDER [CH]; HUBER ULRICH [CH]; SC) 6 October 2005 (2005-10-06)<br>* examples 1b,2,7-9 *<br>* claims 1-16 * | 1-3,6<br><br><br>4,5 | |
| Y | WO 02/36534 A2 (LONZA AG [US]; LEMKE DANIEL WAYNE [US])<br>10 May 2002 (2002-05-10)<br>* claims 1-39 *<br>* example 2 * | 4,5 | TECHNICAL FIELDS SEARCHED (IPC)<br>A61K<br>A61Q |

The present search report has been drawn up for all claims

1

| Place of search | Date of completion of the search | Examiner |
|---|---|---|
| Munich | 24 April 2015 | Wörth, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 8419

This annex lists the patent family members relating to the patent documents cited in the above-mentioned European search report. The members are as contained in the European Patent Office EDP file on
The European Patent Office is in no way liable for these particulars which are merely given for the purpose of information.

24-04-2015

| Patent document cited in search report | Publication date | Patent family member(s) | Publication date |
|---|---|---|---|
| EP 2679616 A1 | 01-01-2014 | AU 2013206511 A1 | 16-01-2014 |
| | | CA 2819831 A1 | 28-12-2013 |
| | | CN 103601882 A | 26-02-2014 |
| | | EP 2679616 A1 | 01-01-2014 |
| | | KR 20140001796 A | 07-01-2014 |
| | | US 2014004061 A1 | 02-01-2014 |
| WO 2014004474 A2 | 03-01-2014 | AU 2013280569 A1 | 22-01-2015 |
| | | AU 2013280572 A1 | 04-12-2014 |
| | | CA 2874898 A1 | 03-01-2014 |
| | | CA 2877345 A1 | 03-01-2014 |
| | | CN 104411292 A | 11-03-2015 |
| | | CN 104411367 A | 11-03-2015 |
| | | EP 2866783 A2 | 06-05-2015 |
| | | EP 2866899 A2 | 06-05-2015 |
| | | TW 201404427 A | 01-02-2014 |
| | | TW 201408330 A | 01-03-2014 |
| | | US 2014004063 A1 | 02-01-2014 |
| | | US 2014004064 A1 | 02-01-2014 |
| | | WO 2014004474 A2 | 03-01-2014 |
| | | WO 2014004477 A2 | 03-01-2014 |
| WO 2011070050 A1 | 16-06-2011 | CN 102656208 A | 05-09-2012 |
| | | EP 2510041 A1 | 17-10-2012 |
| | | US 2012282200 A1 | 08-11-2012 |
| | | WO 2011070050 A1 | 16-06-2011 |
| WO 2005092282 A1 | 06-10-2005 | AU 2005226922 A1 | 06-10-2005 |
| | | CN 1937999 A | 28-03-2007 |
| | | EP 1727515 A1 | 06-12-2006 |
| | | JP 5031551 B2 | 19-09-2012 |
| | | JP 2007535588 A | 06-12-2007 |
| | | KR 20070001199 A | 03-01-2007 |
| | | US 2008081025 A1 | 03-04-2008 |
| | | WO 2005092282 A1 | 06-10-2005 |
| WO 0236534 A2 | 10-05-2002 | AU 2730802 A | 15-05-2002 |
| | | CA 2427854 A1 | 10-05-2002 |
| | | JP 2004531464 A | 14-10-2004 |
| | | US 2002058781 A1 | 16-05-2002 |
| | | WO 0236534 A2 | 10-05-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5869030 A
- US 20120058974 A
- US 6193959 B, Bernasconi
- US 6962692 B
- US 6899866 A
- US 6800274 B
- US 20050036961 A1


### Non-patent literature cited in the description


- **ROKICKI et al.** *Green Chemistry,* 2005, vol. 7, 52